# EUROPÄISCHE PATENTSCHRIFT

(11) **EP 2 462 428 B1**
(45) Veröffentlichungstag und Bekanntmachung des Hinweises auf die Patenterteilung: **14.04.2021**
(21) Anmeldenummer: 10740154.9
(22) Anmeldetag: 10.07.2010
(51) Int. Cl.: B01L 3/00, G01N 33/52, G01N 21/64, G01N 33/487

(54) **OPTISCHER SENSOR MIT LÖSLICHER DECKSCHICHT**
OPTICAL SENSOR WITH SOLUBLE COATING-LAYER
CAPTEUR OPTIQUE AVEC COUCHE DE REVÊTEMENT SOLUBLE

(30) Priorität: 05.08.2009 DE 102009036217
(43) Veröffentlichungstag der Anmeldung: 13.06.2012
(73) Patentinhaber: Sartorius Stedim Biotech GmbH, 37079 Göttingen (DE)
(72) Erfinder: BAUMFALK, Reinhard, 37083 Göttingen (DE); GRELLER, Gerhard, 37085 Göttingen (DE); RIECHERS, Daniel, 93051 Regensburg (DE); LÜDERS, Julia, 31246 Ilsede (DE)
(74) Vertreter: Vigand, Philippe
(86) Internationale Anmeldenummer: PCT/EP2010/004221
(87) Internationale Veröffentlichungsnummer: WO 2011/015270

(56) Entgegenhaltungen:
- EP-A1- 1 795 891
- EP-A2- 0 340 018
- WO-A1-02/056023
- WO-A2-2005/032330
- US-A1- 2002 106 810

## Beschreibung

Die Erfindung betrifft einen durch Bestrahlen sterilisierbaren optischen Sensor, zur Bestimmung mindestens eines Parameters in einem Medium, eine Vorrichtung welche einen solchen optischen Sensor aufweist und ein Verfahren zur Herstellung des Sensors sowie der Vorrichtung. Optische Sensoren werden insbesondere in Einweg-, Misch- und Bioreaktoren bzw. -behältern in der Medizintechnik und Biotechnologie eingesetzt. In diesen und ähnlichen Anwendungsgebieten ist es oft nötig, einen Behälter vor der Benutzung zu sterilisieren. Auf dem Gebiet der Einwegprodukte hat sich eine Sterilisation mittels Strahlung, insbesondere Gamma-Strahlung, bewährt, welche jedoch schädlich für optische Sensoren ist. Dies betrifft insbesondere auf porösen Matrizes basierende optische Sensoren, wie beispielsweise fluoreszenzbasierte pH-Sensoren. Daher wird für derartige Sensoren ein effektives Schutzsystem benötigt, das zugleich kostengünstig realisiert werden kann

Die EP 1 795 891 A1 offenbart einen optischen Sensor zur Messung einer Reihe von Analyten in einer wässrigen Probe. Der Sensor ist ein mehrschichtiges Testelement, das auf der der Probe zugewandten Seite mit einer Deckschicht überzogen ist. Die Deckschicht besteht aus hydrophilen Polymeren die das Ausspreiten der Probe begünstigen.

Aus WO 02/056023 A1 und DE 10,051,220 A1 sind optische Sensoren zur Messung mindestens eines Parameters in einer Probe bekannt. Diese Sensoren basieren auf einer Vorrichtung zur Anregung der Fluoreszenz eines in einem Probengefäß bzw. Reaktor in einer Matrix immobilisierten Analyt-sensitiven Fluoreszenzfarbstoffes, der in zumindest indirektem Kontakt zur Probe steht, und einer Auswertungsvorrichtung für das resultierende Fluoreszenzantwortsignal. Die Auswertung bzw. die Bestimmung der Analyt-Konzentration kann hierbei sowohl durch die Verwertung der Fluoreszenzabklingzeit als auch der Fluoreszenzintensität erfolgen. Nachteilig ist, dass derartige pH-Sensorpatches, basierend auf einer hydrophilen Trägermatrix, wie z.B. imprägnierten Papieren oder Sol-Gel-Matrizes, bei einer Strahlen-Sterilisation dosisabhängig geschädigt werden. Es verringern sich sowohl die Intensität der Fluoreszenz des Farbstoffs bzw. der Farbstoffe, als auch die Sensitivität des Sensorpatches gegenüber der Messgröße.

Aus US 7,390,462 B2 ist ein Sensor bekannt, bei dem der Fluoreszenzfarbstoff in einer hydrophilen Matrix immobilisiert vorliegt. Hierbei wird ein Sensor mit dem pH-sensitiven Fluoreszenzfarbstoff MA-HPDS in einem Hydrogel vorliegend beansprucht. Auch hierbei ist nachteilig, dass derartige hydrophile optische Sensoren bei einer Sterilisation mit Gammastrahlung dosisabhängig geschädigt werden. Eine solche Strahlung wird insbesondere in der Labortechnologie bei Behältern aus Polymeren angewendet. Es verringern sich sowohl die Intensität der Fluoreszenz des Farbstoffs bzw. der Farbstoffe, als auch die Sensitivität des Sensors gegenüber der Messgröße. Eine besonders starke Schädigung eines solchen Sensorpatches tritt auf, wenn er während der Strahlen-Sterilisation in Kontakt mit einem größeren Volumen an Luft, bzw. auch mit üblichen Schutzgasen wie z.B. Stickstoff oder Argon steht. Bei der Strahlen-Sterilisation werden die Gase teilweise ionisiert, bzw. es entstehen freie Radikale. Diese Radikale reagieren beispielsweise bei der Sterilisation eines gasgefüllten Polymer-Beutels an den Wänden oder auch an den Sensoroberflächen ab. Auf porösen, hydrophilen Matrizes basierende Sensoren sind hierfür besonders anfällig, da die Sensorchemie prinzipbedingt an der Oberfläche, bzw. inneren Oberfläche der Matrix immobilisiert vorliegen muss, damit die zu vermessende Probe mit der Sensorchemie in Kontakt kommen kann. Das Ausmaß der Schädigung hängt einerseits von der Bestrahlungsdosis und andererseits vom Verhältnis der Oberfläche zum Volumen des bestrahlten, den Sensorpatch enthaltenden, Behälters ab. Dieses Verhältnis bestimmt die Anzahl an Ionen bzw. Radikalen welche den Sensorpatch, bzw. die darin enthaltene Sensorchemie schädigen.

Aus DE 10 2009 003 971.6 A1 ist ein optischer Sensor zur Messung mindestens eines Parameters bekannt, welcher porös mit einer Edelmetallschicht überzogen ist, womit eine Abreaktion von reaktiven Ionen an der Edelmetallschicht erreicht wird. Hierbei ist jedoch nachteilig, dass sich eine derartige Beschichtung technisch und mechanisch aufwendig gestaltet und mit hohen Kosten verbunden ist, was insbesondere im Bereich von Einwegprodukten zu vermeiden ist.

Der vorliegenden Erfindung liegt daher die Aufgabe zugrunde, zum einen einen optischen Sensor sowie eine Vorrichtung damit zu schaffen, womit die Empfindlichkeit des optischen Sensors gegenüber Strahlung, insbesondere Gammastrahlung, herabgesetzt ist, und welche kostengünstig und einfach realisierbar ist, sowie zum anderen ein Verfahren zu ihrer Herstellung anzugeben.

Die erste Aufgabe wird durch einen optischen Sensor gemäß Anspruch 1 gelöst. Der erfindungsgemäße optische Sensor misst einen oder mehrere Parameter in einem Medium. Im Wesentlichen weist der optische Sensor drei übereinander gelagerte Schichten auf. Als Basisschicht dient ein transparenter Träger welcher von Anregungslicht durchdringbar ist. Auf diesem sitzt eine Matrix, welche mindestens einen Fluoreszenzfarbstoff enthält. Zum Schutz der letztgenannten Schicht ist diese mit einer löslichen Schicht überzogen.

Erfindungsgemäß besteht die lösliche Schicht aus einem/einer oder mehreren Zucker(n), anorganische(n) Salz(en), Aminosäure(n), Glycerin, Polyvinylalkohol(en), Peptid(en), Protein(en) oder einer Kombination daraus. Die lösliche Schicht löst sich erst durch Kontakt mit einem zugegebenem wässrigen Medium in diesem auf. Die Empfindlichkeit gegenüber bei der Gamma-Bestrahlung entstehenden Reaktivteilchen ist stark herabgesetzt. Hierdurch wird ein besseres Signal-Rausch-Verhältnis, sowie eine allgemein höhere Sensitivität des optischen Sensors gegenüber seiner Messgröße erreicht. Zugleich kann beispielsweise eine Anregung oder Inhibition bestimmter Prozesse in einem Zellkultur- oder Fermentationsmedium durch die gelöste Substanz erreicht werden. Die lösliche Schicht kann zusätzlich eine Sicherheits- und Stabilisierungsfunktion gegenüber der sich darunter befindlichen Sensorchemie in Form von der den mindestens einen Fluoreszenzfarbstoff enthaltenden Matrix während beispielsweise des Transports erfüllen.

Gemäß einer bevorzugten Ausführungsform der Erfindung ist der optische Sensor durch Strahlung unter Erhalt seiner Funktionsfähigkeit sterilisierbar. Die Empfindlichkeit gegenüber einer Sterilisierung, beispielsweise mittels ionisierender Strahlung, Gammastrahlung, UV-C-, Beta- oder Elektronenstrahlung ist durch die lösliche Schicht stark herabgesetzt. Die durch die Strahlung gebildeten reaktiven Teilchen in der den optischen Sensor umgebenden Gasphase reagieren nicht mehr mit der Matrix und dem Fluoreszenzfarbstoff bzw. den Fluoreszenzfarbstoffen selbst. Somit wird ein besseres Signal-Rausch-Verhältnis, sowie eine allgemein höhere Sensitivität des optischen Sensors gegenüber seiner Messgröße erreicht. Die Messgrößen können hierbei z.B. der pH-Wert, die Gelöstsauerstoffkonzentration oder andere Parameter sein.

Gemäß einer weiteren bevorzugten Ausführungsform der Erfindung ist die Matrix des optischen Sensors hydrophil. Besondere Ausführungen können eine Sol-Gel-Matrix oder ein Hydrogel sein, in welchem der Fluoreszenzfarbstoff immobilisiert vorliegt. Die hydrophile Eigenschaft ist besonders für optische Sensoren nötig, deren in der Matrix eingebettete Fluoreszenzfarbstoff für ein wässriges Medium zugänglich sein muss. Optische Sensoren, welche eine hydrophile Matrix aufweisen sind naturgemäß äußerst sensibel gegenüber einer Sterilisierung, z.B. mittels Gammastrahlung. Die lösliche Schicht schützt wirkungsvoll gegen diese Einflüsse.

Gemäß einer weiteren besonders bevorzugten Ausführungsform der Erfindung ist die Matrix des optischen Sensors porös.

Gemäß einer weiteren bevorzugten Ausführungsform kann das zu vermessende Medium die lösliche Schicht des optischen Sensors auch teilweise durchdringen. Die lösliche Schicht muss nicht als Ganzes aufgelöst werden, da eine Durchdringung dieser, und somit ein Kontaktieren der Matrix durch das Medium möglich ist. Eine lösliche Schicht, welche zu einem Teil unlösliche Substanzen beinhaltet, kann somit als zusätzliche poröse Schutzmatrix über der Matrix liegen.

Nach einer besonders bevorzugten Ausführungsform der Erfindung weist die gegenüber dem zu vermessenden Medium lösliche Schicht des optischen Sensors eine Dicke von 10 nm bis 2 mm auf.

Besonders vorteilhaft erweist sich hierbei ein Restfeuchtegehalt der löslichen Schicht von 5 - 10 %. Somit ist ein sicherer Schutz während der Sterilisation und ein einfaches Auflösen der löslichen Schicht erreichbar.

In einer weiteren vorteilhaften Ausgestaltung der Erfindung ist die gegenüber dem zu vermessenden Medium lösliche Schicht gegenüber dem optischen Sensor inert. Dadurch ist eine störungsfreie korrekte Funktion des optischen Sensors gewährleistet.

In einer vorteilhaften Ausgestaltung der Erfindung ist die gegenüber dem zu vermessenden Medium lösliche Schicht inert gegenüber dem zu vermessenden Medium selbst. Sie löst sich durch dieses auf, bedingt aber keine weiteren Produkte

Nach einer weiteren bevorzugten Ausführungsform ist die gegenüber dem zu vermessenden Medium lösliche Schicht durch Mikroorganismen oder Zellkulturen metabolisierbar. Somit ist ein Eintrag von unerwünschten Stoffen, bzw. ein Überangebot an zu metabolisierenden Stoffen vor Prozessen durch entsprechende Kalkulation unterbindbar. Auch ein umgekehrter Effekt ist ausnutzbar, indem gelöste Stoffe als Inhibitor für bestimmte Prozesse fungieren.

In einer weiteren vorteilhaften Ausgestaltung der Erfindung ist die gegenüber dem zu vermessenden Medium lösliche Schicht des optischen Sensors der Vorrichtung gegenüber dem optischen Sensor inert, wodurch eine störungsfreie korrekte Funktion des optischen Sensors gewährleistet ist.

In einer vorteilhaften Ausgestaltung der Erfindung ist die gegenüber dem zu vermessenden Medium lösliche Schicht des optischen Sensors der Vorrichtung inert gegenüber dem vermessenden Medium selbst. Sie löst sich durch dieses auf, bedingt aber keine weiteren Produkte.

Nach einer weiteren bevorzugten Ausführungsform ist die gegenüber dem zu vermessenden Medium lösliche Schicht des optischen Sensors der Vorrichtung durch Mikroorganismen oder Zellkulturen metabolisierbar. Somit ist ein Eintrag von zusätzlichen Stoffen, bzw. ein Überangebot an zu metabolisierenden Stoffen vor Prozessen durch entsprechende Kalkulation unterbindbar. Auch ein umgekehrter Effekt ist ausnutzbar, indem gelöste Stoffe als Inhibitor für bestimmte Prozesse fungieren.

Bei der Herstellung eines erfindungsgemäßen optischen Sensors werden im Wesentlichen zwei Schritte angewendet. Zum einen das Aufbringen einer mindestens einen Fluoreszenzfarbstoff aufweisenden Matrix auf eine transparente Trägerschicht und zum anderen das Aufbringen einer wasserlöslichen Schicht auf die Matrix. Die lösliche Schicht ist hierbei ein/eine(n) oder mehrere Zucker, anorganische(s) Salz(e), Aminosäure(n), Peptid(e), Protein(e), Glycerin, Polyvinylalkohol(e) enthaltende Lösung sein.

Bei einem weiteren bevorzugtem Verfahren zur Herstellung einer einen optischen Sensor zum Bestimmen mindestens eines Parameters in einem Medium aufweisenden Vorrichtung, bei der die Funktionsfähigkeit des optischen Sensors nach einer Sterilisation durch Bestrahlen erhalten bleibt, werden im Wesentlichen folgende Verfahrensschritte angewendet: Zuerst wird der optische Sensor mit der Vorrichtung zur Aufnahme des Mediums kombiniert; daraufhin wird die Vorrichtung mittels Strahlung bestrahlt.

Bei einem bevorzugtem Verfahren gemäß der Erfindung wird das Bestrahlen der Vorrichtung mittels ionisierender Strahlung, beispielsweise durch Gammastrahlung, UV-C-, Beta- oder Elektronenstrahlung, vollzogen.

Bei einem weiteren besonders bevorzugtem Verfahren wird für die Vorrichtung ein Behälter aus Kunststoff mit zumindest teilweise flexiblen Wänden verwendet. Derartige Behälter sind kostengünstig herzustellen. Im Falle von Einwegprodukten entfällt zudem eine zeit- und kostspielige Reinigung. Ein oder mehrere optische Sensoren können in einen Behälter aus Kunststoff implementiert werden. Die Sensormessleistung bleibt trotz einer nötigen Sterilisation des Kunststoffbehälters mit Strahlung erhalten.

Die Erfindung soll an dem nachstehenden Ausführungsbeispiel näher erläutert werden.

### Beispiel:

Die Sensorchemie von drei pH-Sensoren (HP8, Presens GmbH, Regensburg) auf Polycarbonat-Kappe, wird mit 10µl Glycerin (99%, Sigma-Aldrich, Schnelldorf) imprägniert, 3 weitere Sensoren des gleichen Typs werden in gleicher Art mit 10µl einer 50%igen Glucose-Lsg. (Glucose-Monohydrat, Sigma-Aldrich, Schnelldorf) behandelt. Die sechs behandelten Sensoren werden zusammen mit drei unbehandelten Sensoren des gleichen Typs über Nacht im Trockenschrank bei 40°C getrocknet. Die insgesamt neun Sensoren werden in einen Kunststoffbehälter (Cultibag RM, Sartorius Stedim Biotech GmbH, Göttingen/Volumen: 10 L) eingebracht und so befestigt dass die Sensorchemie dem Mittelpunkt des Behälters zugewandt ist. Die Vorrichtung wird in einem schwarzen, lichtundurchlässigen PE-Beuteln verpackt. Daraufhin erfolgt eine Gamma-Bestrahlung bei 28,0 kGy (Co60-Quelle, Beta-Gamma-Service GmbH & Co. KG, Wiehl). Nach der Bestrahlung werden die Sensoren aus dem Behälter entnommen und mit einem Transmitter (pH-1 mini, Presens GmbH, Regensburg) in Puffern mit pH-Werten von 6,0 und 8,0 vermessen. Die Empfindlichkeit und damit die Güte der Sensoren entspricht der Differenz der Phasen bei pH 6,0 und pH 8,0. Werte hierzu sind in der folgenden Tabelle angegeben.

| Sensor, vorbehandelt mit | Dosis Gamma [kGy] | Δ Phase [°] (Mittelwert aus je 3 Sensoren) |
|---|---|---|
| Unbehandelt | 28,0 | 19 |
| 10 µl Glycerin | 28,0 | 20 |
| 10 µl Glucose-Lsg. 50% | 28,0 | 23 |

Wie aus vorstehender Tabelle ersichtlich ist, ist die Empfindlichkeit der Imprägnierten Sensoren höher als die unbehandelter Sensoren.

## Patentansprüche

1. Optischer Sensor zur Bestimmung mindestens eines Parameters in einem Medium aus mindestens einer Matrix, die wenigstens einen Fluoreszenzfarbstoff enthält, welche von einem transparenten Träger getragen ist und die auf der dem Medium zugewandten Seite eine gegenüber dem zu vermessenden Medium lösliche Schicht aufweist, wobei die gegenüber dem zu vermessenden Medium lösliche Schicht aus einem/einer oder mehreren Zucker(n), anorganische(n) Salz(en), Aminosäure(n), Glycerin, Peptid(en), Protein(en) oder einer Kombination daraus besteht.

2. Optischer Sensor nach Anspruch 1, wobei die Matrix hydrophil ist.

3. Optischer Sensor nach einem der vorangehenden Ansprüche, wobei die Matrix porös ist.

4. Optischer Sensor nach einem der vorangehenden Ansprüche, wobei die Matrix eine Sol-Gel Matrix oder ein Hydrogel ist.

5. Optischer Sensor nach einem der vorangehenden Ansprüche, wobei die gegenüber dem zu vermessenden Medium lösliche Schicht eine Dicke von 10 nm bis 2 mm aufweist.

6. Optischer Sensor nach einem der vorangehenden Ansprüche, wobei der Restfeuchtegehalt der löslichen Schicht 5-10 % beträgt.

7. Optischer Sensor nach einem der vorangehenden Ansprüche, wobei die gegenüber dem zu vermessenden Medium lösliche Sicht gegenüber dem optischen Sensor inert ist.

## Claims

1. An optical sensor for determining at least one parameter in a medium consisting of at least one matrix, which contains at least one fluorescent dye which is carried by a transparent support and which has a soluble layer on the side facing the medium vis-à-vis the medium to be measured, wherein the soluble layer vis-à-vis the medium to be measured consists of one or more sugar(s), inorganic salt(s), amino acid(s), glycerin, peptide(s), protein(s), or a combination thereof.

2. The optical sensor according to claim 1, wherein the matrix is hydrophilic.

3. The optical sensor according to any of the preceding claims, wherein the matrix is porous.

4. The optical sensor according to any of the preceding claims, wherein the matrix is a sol-gel matrix or a hydrogel.

5. The optical sensor according to any of the preceding claims, wherein the soluble layer vis-à-vis the medium to be measured has a thickness of from 10 nm to 2 mm.

6. The optical sensor according to any of the preceding claims, wherein the residual moisture content of the soluble layer is from 5 to 10%.

7. The optical sensor according to any of the preceding claims, wherein the soluble layer vis-à-vis the medium to be measured is inert vis-à-vis the optical sensor.

## Revendications

1. Capteur optique pour la détermination d'au moins un paramètre dans un milieu, composé d'au moins une matrice qui contient au moins une substance fluorescente qui est supportée par un support transparent et qui présente une couche soluble par rapport au milieu à mesurer sur la face tournée vers le milieu, dans lequel la couche soluble par rapport au milieu à mesurer est constituée d'un ou de plusieurs sucre(s), sel(s) inorganique(s), acide(s) aminé(s), glycérine, peptide(s), protéine(s) ou d'une combinaison de ceux-ci.

2. Capteur optique selon la revendication 1, dans lequel la matrice est hydrophile.

3. Capteur optique selon l'une des revendications précédentes, dans lequel la matrice est poreuse.

4. Capteur optique selon l'une des revendications précédentes, dans lequel la matrice est une matrice sol-gel ou un hydrogel.

5. Capteur optique selon l'une des revendications précédentes, dans lequel la couche soluble par rapport au milieu à mesurer présente une épaisseur de 10 nm à 2 mm.

6. Capteur optique selon l'une des revendications précédentes, dans lequel la teneur en humidité résiduelle de la couche soluble est de 5 à 10 %.

7. Capteur optique selon l'une des revendications précédentes, dans lequel la couche soluble par rapport au milieu à mesurer est inerte vis-à-vis du capteur optique.
